# EUROPEAN PATENT APPLICATION

(11) **EP 2 767 226 A1**
(43) Date of publication of application: **20.08.2014**
(21) Application number: 14155281.0
(22) Date of filing: 14.02.2014
(51) Int. Cl.: A61B 5/00

(54) **Medical measurement device and measurement system**

(30) Priority: 18.02.2013 JP 2013028907
(71) Applicant: ARKRAY, Inc., Kyoto-shi, Kyoto 601-8045 (JP)
(72) Inventor: Nakagawa, Takashi, Kyoto-shi, Kyoto 602-0008 (JP)
(74) Representative: MacDougall, Alan John Shaw

(57) **Abstract**

The present invention can prevent communication from being set erroneously while simplifying a communication setting operation between a medical measurement device and an external device.

A measurement device 10 includes: a measurement portion 3 that acquires measurement data; a setting portion 41 that specifies an external device 14 as a transmission destination of the measurement data based on a signal transmitted from the external device 14, and allows communication with the external device 14 without receiving input of authentication information; and a transmission and reception portion 42 that transmits measurement data to the external device 14. The setting portion 41 causes the transmission and reception portion 42 to transmit identification information of the measurement device 10 to the external device 14, and further to transmit confirmation information thereto in addition to the identification information, wherein the confirmation information is used by the external device 14 to decide whether or not the external device 14 can handle the measurement data transmitted from the measurement device 10.

## Description

### BACKGROUND OF THE INVENTION

### 1. Field of the Invention

The present invention relates to a technique for acquiring information about a subject body and processing the information.

### 2. Description of Related Art

For example, most of medical measurement devices such as a portable blood glucose meter have a function of transmitting measurement data of a subject body obtained by measurement to another device. For example, JP 2000-29972 A and JP 2009-181593 A disclose a system of transmitting data of blood glucose levels recorded in a clinical analysis device to a computer system, together with an identification password. Further, JP 2003-144394 A discloses a case of transmitting control log information to a server simultaneously with biological information from a patient terminal and analyzing the information in the server.

Recently, along with the spread of communication devices, setting of data communication between a medical measurement device and a user's communication device (e.g., smartphone, tablet terminal, etc.) is increasing. For example, a pairing operation sometimes is required that allows wireless communication between a measurement device and an external device. At this time, a setting operation of a measurement device by a user is demanded to be as simple as possible. However, when the setting operation for communication with an external device is facilitated, unauthorized access from the outside may increase and erroneous communication with another device may be established. The above-described conventional techniques do not disclose measures for solving such problems in the communication setting between a measurement device and an external device.

Conventionally, when establishing the communication between a measurement device and an external device, at least one of the measurement device and the external device requires a user to input authentication information for authenticating the other device. The authentication information is used at the time of establishing communication between devices so as to confirm before establishing the communication that the other device is a communication partner. For example, in order to facilitate an operation at the time of establishing communication, if both of the measurement device and the external device do not require input of authentication information, communication may be established between erroneous devices. Particularly in a medical field, it is assumed that a plurality of users perform communication setting between their own measurement devices and external devices simultaneously in the same place. To cope with this situation, a communication setting operation of a measurement device with respect to an external device should be facilitated, while avoiding establishment of communication with an erroneous external device. The present application discloses a technique of preventing communication from being set erroneously while simplifying a communication setting operation between a medical measurement device and an external device.

### SUMMARY OF THE INVENTION

A medical measurement device disclosed by the present application is a medical measurement device that acquires information about a subject body, including: a measurement portion that acquires measurement data obtained by measurement; a setting portion that specifies an external device as a transmission destination of the measurement data based on a signal transmitted from the external device, and records information of the specified external device, thereby allowing communication with the external device without receiving input of authentication information both in the external device and the measurement device; and a transmission portion that transmits the measurement data to the external device specified by the setting portion. The setting portion causes the transmission portion to transmit identification information of the measurement device for enabling mutual communication with respect to the external device as a transmission source of the received signal, and further to transmit confirmation information thereto in addition to the identification information at or after the transmission of the identification information, wherein the confirmation information is used by the external device to decide whether or not the external device can handle the measurement data transmitted from the measurement device.

According to the disclosure of the present application, it is possible to prevent communication from being set erroneously while simplifying a communication setting operation between a medical measurement device and an external device.

### BRIEF DESCRIPTION OF THE DRAWINGS

[FIG. 1] FIG. 1 is a functional block diagram showing an exemplary configuration of a measurement system in an embodiment.
[FIG. 2] FIG. 2 is a sequence diagram showing an exemplary operation and an exemplary screen at the time of establishment of communication between a measurement device and an external device.
[FIG. 3] FIG. 3 is a flowchart showing exemplary setting processing by a setting portion of the measurement device.
[FIG. 4] FIG. 4 is a flowchart showing exemplary setting processing by a measurement device determination portion of the external device.
[FIG. 5] FIG. 5 is a perspective view showing an exemplary blood glucose meter that can be adopted as a measurement device.
[FIG. 6] FIG. 6 shows a measurement device and an external device as one example.
[FIG. 7] FIG. 7 shows a measurement device and an external device as another example.

### DETAILED DESCRIPTION OF THE INVENTION

### (Exemplary Configuration of System)

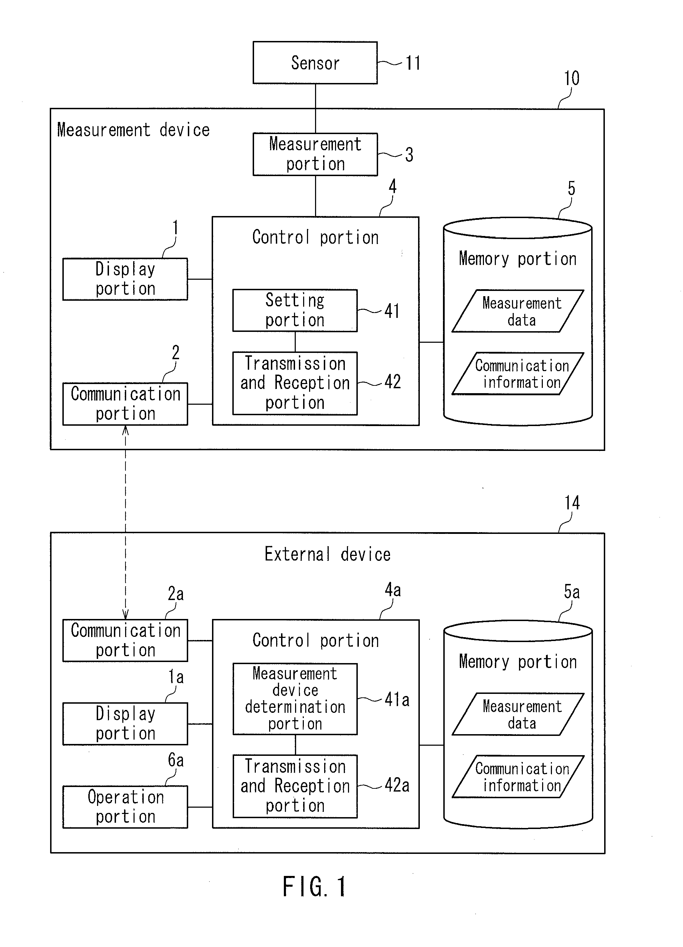

FIG. 1 is a functional block diagram showing an exemplary configuration of a measurement system in an embodiment of the present invention. The measurement system shown in FIG. 1 includes a measurement device 10 and an external device 14. The measurement device 10 acquires information about a subject body obtained by measurement, and records the information. The external device 14 receives measurement data from the measurement device 10, and processes the data.

As one example, the present embodiment describes a case where the measurement device 10 is a portable blood glucose meter (glucometer) used for measuring a user's own glucose level, and the external device 14 is a general-purpose computer such as a personal computer, a smartphone and a tablet terminal of a user. However, the measurement device 10 and the external device 14 are not limited to particular devices.

### (Exemplary Configuration of Measurement Device 10)

In the example shown in FIG. 1, the measurement device 10 is provided with a display portion 1, a communication portion 2, a measurement portion 3, a control portion 4, and a memory portion 5. The measurement portion 3 acquires measurement data obtained by measurement. For example, the measurement portion 3 provides the control portion 4 with measurement values based on information obtained via a sensor 11. The control portion 4 includes a setting portion 41 and a transmission and reception portion 42. The setting portion 41 specifies an external device as a transmission destination of measurement data based on a signal transmitted from the external device, and records information of the specified external device, thereby allowing communication with the external device. At this time, the setting portion 41 brings the measurement device 10 into a state communicable with the external device without receiving input of authentication information. The transmission and reception portion 42 includes a transmission portion that transmits measurement data to the external device specified by the setting portion 41. Further, the transmission and reception portion 42 can receive signals and the like from the external device. Other than this explanation, the transmission portion and the reception portion may be independent from each other.

The setting portion 41 causes the transmission and reception portion 42 to transmit identification information of the measurement device for enabling mutual communication, with respect to the external device as a transmission source of the received signal. Further, the setting portion 41 causes the transmission and reception portion 42 to transmit confirmation information with respect to the external device in addition to the identification information at or after the transmission of the identification information. The confirmation information is used by the external device to decide whether or not the external device can handle the measurement data transmitted from the measurement device 10.

In the above-described configuration, the measurement device 10 receives a signal from the external device, records information of the external device, and transmits identification information of the measurement device to the external device, thereby simplifying a user's operation when establishing communication with the external device. For example, the above-described configuration allows bidirectional communication without reception of authentication information from a user both in the external device and the measurement device. Further, the measurement device 10 transmits confirmation information to the external device at or after the establishment of communication with the external device as a transmission destination of measurement data. Thus, the external device can decide using the confirmation information whether or not erroneous communication is set between the external device and the measurement device 10. In other words, the transmission of confirmation information from the measurement device 10 to the external device makes it possible to detect an erroneous, not-intended communication setting.

The confirmation information may include information specific to the measurement device 10, for example. Examples of the confirmation information include a product serial number of the measurement device 10, a user's telephone number, a preset password, etc. The confirmation information transmitted from the measurement device 10 is, for example, compared with authentication information input from a user in the external device. Thus, the propriety of the established communication can be judged based on the comparison result.

The setting portion 41 can be configured to cause the transmission and reception portion 42 to transmit confirmation information to the external device, after specifying the external device as a transmission destination of measurement data and allowing communication with the external device. In this manner, by establishing communication with the external device and thereafter transmitting confirmation information to the external device, the measurement device 10 can establish communication with a simple operation and thereafter judge the propriety of the established communication. For example, the setting portion 41 also may be configured to cause the transmission and reception portion 42 to transmit confirmation information to the external device together with measurement data, after specifying the external device as a transmission destination of measurement data and establishing communication with the external device. Thus, the external device can judge whether or not the transmitted measurement data is data from the measurement device under legitimately established communication.

The setting portion 41 can cause the display portion 1 of the measurement device 10 to display confirmation information, thereby allowing a user to know confirmation information of the measurement device 10. When the measurement device 10 and the external device in communication are placed close to each other, a user can check confirmation information displayed on the measurement device 10 and input it to the external device. The external device can decide the propriety of the established communication based on whether or not the confirmation information input from a user matches the confirmation information transmitted from the measurement device 10. Thus, it is possible to confirm that the communication established between the external device and the measurement device 10 is communication intended by a user. In this case, the setting portion 41 can cause the display portion 1 to display confirmation information at the timing of transmitting confirmation information.

The setting portion 41 can cause the transmission and reception portion 42 to transmit confirmation information to the external device in association with measurement data to be transmitted by the transmission and reception portion 42. The confirmation information can be information to be used by the external device to decide the usability of measurement data in the external device. Thus, on the measurement device 10 side, it is possible to easily control the usability of measurement data in the external device. For example, the setting portion 41 can generate, as confirmation information, a confirmation code and a control instruction that allows expansion of data by the confirmation code, and associate the information with transmission data to be transmitted.

The setting portion 41 can cancel the state communicable with the external device when receiving a notification from the external device that the external device has judged the measurement data as not handleable based on the confirmation information. Thereby, it is possible to cancel the erroneously established communication setting with the external device. For example, the external device can judge whether the confirmation information transmitted from the measurement device 10 matches the confirmation information input from a user, and cause the transmission and reception portion 42 to notify the measurement device 10 of the result. If the measurement device 10 receives the notification from the external device that they do not match each other, the setting portion 41 can cancel the state communicable with the external device. Examples of the cancellation processing include processing in the measurement device 10 of deleting information of the external device registered as a transmission destination of measurement data, and the like.

### (Exemplary Configuration of External Device 14)

In the example shown in FIG. 1, the external device 14 is provided with a communication portion 2a, a display portion 1a, an operation portion 6a, a control portion 4a, and a memory portion 5a. The control portion 4a includes a measurement device determination portion 41a, and a transmission and reception portion 42a. The measurement device determination portion 41a specifies a measurement device as a transmission source of measurement data based on a signal from the measurement device, and allows communication with the measurement device. The transmission and reception portion 42a includes a reception portion that receives measurement data from the measurement device specified by the measurement device determination portion 41a. Further, the transmission and reception portion 42a can transmit signals and the like to the measurement device. Other than this explanation, the transmission portion and the reception portion may be independent from each other.

The measurement device determination portion 41a decides whether or not the external device can handle the measurement data transmitted from the measurement device based on confirmation information transmitted from the measurement device. Thereby, it is possible to establish communication between the external device 14 and the measurement device 10 by an easy operation, while avoiding erroneous establishment of communication.

In the present example, the communication portion 2 of the measurement device 10 and the communication portion 2a of the external device 14 are communication modules that allow wireless communication with an external device. The transmission form by the communication portion 2 is not particularly limited. The communication portion 2 can adopt a near field wireless communication technology such as Bluetooth (registered trademark), RFID, Zigbee (registered trademark), wireless LAN and UWB, or an infrared communication technology. Through the communication portion 2, the setting portion 41 can receive signals from the external device 14 and transmit confirmation information to the external device 14, and the transmission and reception portion 42 can transmit measurement data to the external device.

The display portion 1 of the measurement device 10 and the display portion 1a of the external device 14 are image display means, and can be composed of a liquid crystal display, for example. Note here that an information output means with respect to a user is not limited to the configurations of the display portions 1 and 1a. For example, the information output means may either display images on an external display connected to the measurement device 10 or the external device 14, or output sound. The operation portion 6a is an information input means to be operated by a user, and can include an input device such as a dial key and a direction key Alternatively, the display portion and the operation portion can be formed integrally as a touch panel (display panel with touch sensor).

The measurement portion 3 may either generate measurement values by performing measurement, or acquire measurement values from the outside. For example, the measurement portion 3 can receive voltage signals indicating measurement results from the sensor 11, subject them to AD conversion, generate measurement data indicating the measurement values, and supply the data to the control portion 4. Alternatively, the measurement portion 3 may receive measurement values from an external device such as the sensor 11, and pass them to the control portion 4.

Note here that a subject to be measured by the measurement portion 3 is not particularly limited. In other words, the measurement portion 3 includes any kind of configuration having a function of generating or acquiring measurement values that can be recorded in association with measurement times. As one example, the measurement portion 3 generates measurement values based on signals or data obtained from the sensor 11 that is attachable/detachable to/from the measurement device 10.

The measurement device 10 can be composed of one computer, or a plurality of computers. The setting portion 41 and the transmission and reception portion 42 of the control portion 4 can be realized by execution of a predetermined program by a processor provided in the computer. For example, a microcontroller can be incorporated in the measurement device 10. As one example, such a microcontroller can include a core processor that constitutes the measurement portion 3, which generates measurement data by subjecting voltage signals transmitted from the sensor 11 to the AD conversion, and the control portion 4.

The external device 14 also can be composed of one computer, or a plurality of computers. The measurement device determination portion 41a and the transmission and reception portion 42a of the control portion 4a can be realized by execution of a predetermined program by a processor provided in the computer.

A recording device such as a memory and a hard disk can be used as the memory portion 5 of the measurement device 10 and the memory portion 5a of the external device 14. The memory portions 5 and 5a may be housed in the measurement device 10 or the external device 14, or may be provided outside independently from the measurement device 10 or the external device 14.

The following also are included as an embodiment of the present invention: a program for causing a computer to function as the setting portion 41 and the transmission and reception portion (transmission portion) 42, or a program for causing a computer to function as the measurement device determination portion 41a and the transmission and reception portion (reception portion) 42a, and a non-transitory recording medium that records these programs. Moreover, a method for a computer to execute these programs also is included as an embodiment of the present invention.

For example, the following program also is one embodiment of the present invention:
a program for activating a computer as a measurement device, the program causing the computer to execute:
   processing of acquiring measurement data obtained by measurement;
   setting processing of specifying an external device as a transmission destination of the measurement data based on a signal transmitted from the external device, and recording information of the specified external device, thereby allowing communication with the external device without receiving input of authentication information; and
   transmission processing of transmitting the measurement data to the external device specified by the setting processing,
   wherein the setting processing includes:
      transmitting identification information of the measurement device for enabling mutual communication with respect to the external device as a transmission source of the received signal; and
      further transmitting confirmation information thereto in addition to the identification information at or after the transmission of the identification information,
      wherein the confirmation information is used by the external device to decide whether or not the external device can handle the measurement data transmitted from the measurement device.

Further, the following program also is one embodiment of the present invention:
a program for activating a computer that receives measurement data from a measurement device and processes the data, the program causing the computer to execute:
   measurement device determination processing of specifying a measurement device as a transmission source of the measurement data based on a signal from the measurement device, and allowing communication with the measurement device without receiving input of authentication information; and
   reception processing of receiving the measurement data from the specified measurement device,
   wherein the measurement device determination processing of the external device includes:
      receiving identification information of the measurement device from the measurement device;
      receiving confirmation information therefrom in addition to the identification information at or after the reception of the identification information; and
      deciding whether or not the measurement data transmitted from the measurement device can be handled based on the confirmation information.

### (Exemplary Operation)

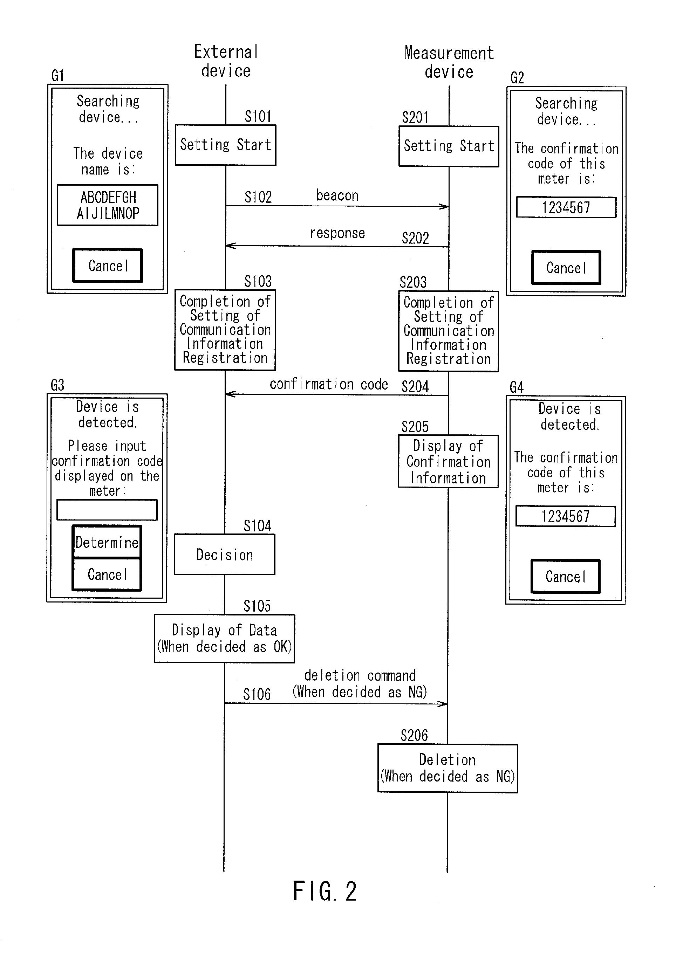

FIG. 2 is a sequence diagram showing an exemplary operation and an exemplary screen at the time of establishment of communication between the measurement device 10 and the external device 14. The example shown in FIG. 2 is a case where the measurement device 10 and the external device 14 are paired in wireless communication based on Bluetooth (registered trademark) standards, thereby being communicable with each other. In this example, the external device 14 operates as a master, and the measurement device operates as a slave. Here, "pairing" is one exemplary processing in which the setting portion 41 of the measurement device 10 and the measurement device determination portion 41a of the external device 14 each specify a communication partner for enabling mutual communication. The pairing includes processing of authenticating a communication partner.

In the example shown in FIG. 2, first, both of the external device 14 and the measurement device 10 start processing of specifying and setting a communication partner (called pairing in this example) (S101, S201). For example, a user switches the external device 14 and the measurement device 10 to a pairing mode, thereby starting pairing. G1 shown in FIG. 2 is an exemplary screen in setting start (S101) of the external device 14, and G2 is an exemplary screen in setting start (S201) of the measurement device 10.

The external device 14 transmits a beacon signal to search a peripheral device (S102). The beacon signal can contain identification information of the external device 14. The measurement device 10 that has received the beacon signal transmits a response to the external device 14 (S202). The response can contain identification information of the measurement device 10. The external device 14 that has received the response signal from the measurement device 10 records, in the memory portion 5a, information to be used for communication with the measurement device 10, such as identification information of the measurement device 10 (S103: registration of communication information). Also, the measurement device 10 records, in the memory portion 5, communication information, such as identification information of the external device 14 (S203). The communication between the measurement device 10 and the external device 14 becomes possible via S103 and S203. In other words, pairing is completed.

In the above-described example, the pairing can be completed without user's confirmation or input of identification information and authentication information (e.g., a passkey, an authentication key, a PIN, etc.) both in the external device 14 and the measurement device 10. This becomes possible by using Bluetooth (registered trademark) version 4.0, for example. In this case, before the establishment of communication between the external device 14 and the measurement device 10, the external device 14 and the measurement device 10 do not require input of authentication information so as to confirm that the other device is a connection partner.

Upon completion of pairing, the measurement device 10 transmits a confirmation code (exemplary confirmation information) to the external device 14 (S204). Further, the measurement device 10 displays the confirmation code on the display portion 1 (S205, exemplary screen G4).

In decision processing S104, the external device 14 receives input of a confirmation code from a user (exemplary screen G3), and compares the confirmation code input from the user with the confirmation code transmitted from the measurement device 10. When they match each other, the external device 14 can execute processing of data transmitted from the measurement device 10. For example, it is possible to permit an application of the external device 14 to process data (e.g., display of data (S105) etc.) transmitted from the measurement device 10.

When the confirmation code input by a user does not match the confirmation code received from the measurement device 10, or when there is no input after a certain period of time from the start of reception of input, both of the external device 14 and the measurement device 10 execute processing of invalidating the paired device. For example, the external device 14 transmits a deletion command to the measurement device 10 (S106). By deleting the communication information recorded in the memory portion 5, the measurement device 10 can invalidate the communication setting between the external device 14 and the measurement device 10 (S206). Thereby, even when erroneous pairing is established, it is possible to prevent exchange of data, leakage of personal information, etc.

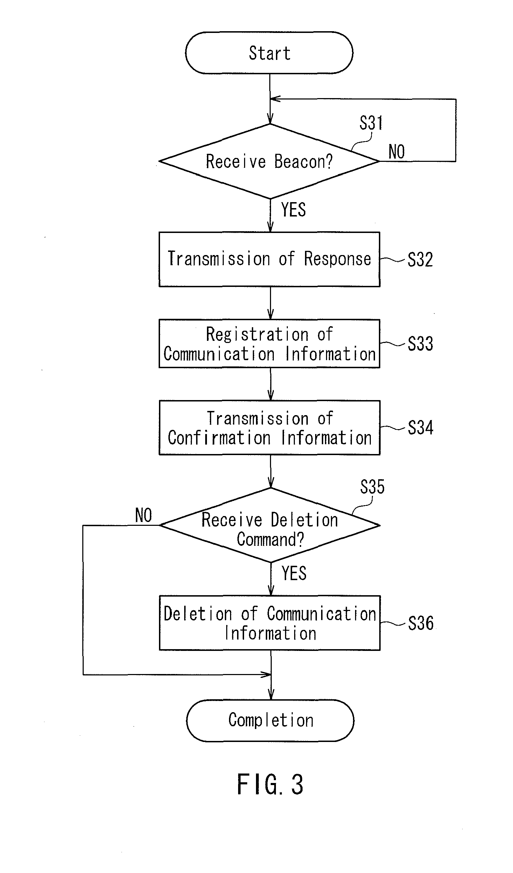

FIG. 3 is a flowchart showing exemplary setting processing by the setting portion 41 of the measurement device 10. The example shown in FIG. 3 shows exemplary processing of the measurement device 10 in the communication setting processing shown in FIG. 2. When the measurement device 10 is switched to a communication setting mode (e.g., pairing mode), the setting portion 41 waits for a beacon signal from a master device (S31). When the transmission and reception portion 42 receives a beacon signal, the setting portion 41 causes the transmission and reception portion 42 to transmit a response signal to the master device (here, the external device 14 as one example) as a transmission source of the beacon signal (S32). The response signal can contain identification information of the measurement device 10.

Upon completion of the transmission of the response signal, the setting portion 41 records, in the memory portion 5, information about the master device contained in the beacon signal received in S31 (S33), thereby allowing communication with the master device. In other words, pairing is established. In S33, communication information to be used for communication with the master device is recorded. Examples of the communication information include identification information of the master device, key information for encryption communication, etc.

The setting portion 41 causes the transmission and reception portion 42 to transmit confirmation information to the master device (S34). For example, the setting portion 41 can cause the transmission and reception portion 42 to transmit confirmation information by adding confirmation information to a header of data to be transmitted to the master device. For example, the setting portion 41 can associate confirmation information with measurement data recorded in the measurement device 10, and cause the transmission and reception portion 42 to transmit the information to the master device together with the measurement data. At that time, the confirmation information can limit the use of the measurement data in the master device. As one example, the setting portion 41 can cause the transmission and reception portion 42 to transmit, together with the confirmation information, a control instruction that allows an application of the master device to use the measurement data only when the confirmation information matches the confirmation information input from a user in the master device. Thus, on the measurement device side, it is possible to control the use of data in the master device.

When the transmission and reception portion 42 receives a deletion command from the master device in reply to the confirmation information (YES in S35), the setting portion 41 judges that pairing is established with an erroneous master device, and deletes the communication information recorded in S33 (S36), thereby canceling the pairing with the master device.

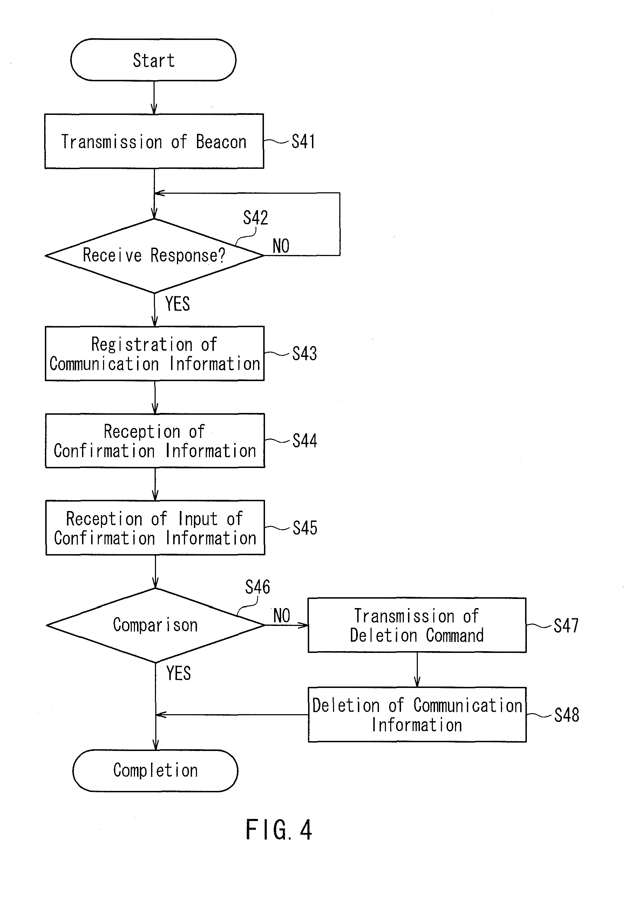

FIG. 4 is a flowchart showing exemplary setting processing by the measurement device determination portion 41a of the external device 14. The example shown in FIG. 4 shows exemplary processing of the external device 14 in the communication setting processing shown in FIG. 2. When the external device 14 is switched to a communication setting mode (e.g., pairing mode), the measurement device determination portion 41a causes the transmission and reception portion 42a to transmit a beacon signal to search a peripheral slave device (S41).

When the transmission and reception portion 42a receives a response signal from the slave device (here, the measurement device 10 as one example) in reply to the beacon signal (YES in S42), the measurement device determination portion 41a records, as communication information in the memory portion 5a, information of the slave device contained in the response signal together with other information necessary for communication (S43), thereby allowing communication with the slave device. In other words, pairing is established. Here, examples of the communication information include identification information of the slave device, key information for encryption communication, etc.

The external device 14 receives confirmation information from the paired slave device (S44). The confirmation information may be contained in a header of data transmitted from the measurement device 10, for example. The measurement device determination portion 41a receives the input of confirmation information from a user (S45). The measurement device determination portion 41a compares the confirmation information received from the slave device in S44 with the confirmation information received from the user in S45 (S46), and causes the transmission and reception portion 42a to transmit a deletion command to the slave device when they do not match each other (S47). Further, the measurement device determination portion 41a deletes the communication information recorded in S43 (S48). Thereby, the pairing can be cancelled both in the external device 14 and the slave device.

Incidentally, also when there is no input after a certain period of time from the start of reception of input of confirmation information in S46, the measurement device determination portion 41a judges that erroneous pairing is established, and can cause the transmission and reception portion 42a to transmit a deletion command (S47). Further, when the confirmation information matches each other (YES) as a result of the comparison in S46, the measurement device determination portion 41a judges that processing of the data transmitted in association with confirmation information is possible, and may proceed to processing of said data in the external device 14. Thereby, based on the confirmation information transmitted from the paired slave device, the measurement device determination portion 41a can judge the usability of data transmitted from the slave device in the external device 14.

The operation of the measurement device 10 and the operation of the external device 14 are not limited to the above-described example. For example, the confirmation information transmitted from the measurement device 10 can be information input from a user in the measurement device 10. In this case, for example, by a user simply inputting certain information both in the measurement device 10 and the external device 14 that is communicable with the measurement device 10, it is possible to prevent establishment of communication between erroneous devices.

It also is possible to adjust the timing of transmitting confirmation information from the measurement device 10 to the external device 14 so that the confirmation information is transmitted at the time of pairing, not after completion of pairing. In this case, the measurement device 10 can decide at the time of pairing whether or not communication with an intended external device becomes possible. Further, the confirmation information may be transmitted together with data as in the above-described example, or may be transmitted alone separately from data.

As a modification example of the processing of judging the usability of data in the external device 14 using confirmation information, the external device 14 can display the confirmation information transmitted from the measurement device 10, and receive input whether or not the displayed confirmation information is information of the measurement device 10 intended by the user. In this case, since the user only needs to input YES or NO, the user's operation with respect to the external device 14 can be facilitated further.

As cancellation processing of a communication setting when the established communication (pairing) between the measurement device 10 and the external device 14 is judged as an erroneous communication, for example, data transmitted from the measurement device 10 can be set so as not to be used by an application of the external device 14, instead of deleting the communication information as in the above-described example. In this case, the external device 14 can request a user to input or confirm confirmation information again if data is transmitted from the erroneously paired measurement device 10.

As described above, in the present embodiment, the communication between the measurement device 10 and the external device 14 is established without the use of authentication information, and the measurement device 10 transmits confirmation information together with data to be transmitted to the external device 14 after establishment of communication. The confirmation information is information to control the usability of transmitted data in the external device 14. Hence, in the external device 14, it is possible to judge the legitimacy of data from the measurement device 10 and execute appropriate processing based on the judgment results. In other words, the present embodiment can solve disadvantages due to simplification of the communication setting operation between the measurement device and the external device, by devising the control of information from the measurement device. Thereby, the risk of transmitting data from the measurement device 10 to an erroneous external device can be reduced, while simplifying the communication setting operation.

### (Exemplary Application to Blood Glucose Meter)

The following describes a case of applying the present invention including the above-described embodiment to a measurement system of a blood glucose meter.

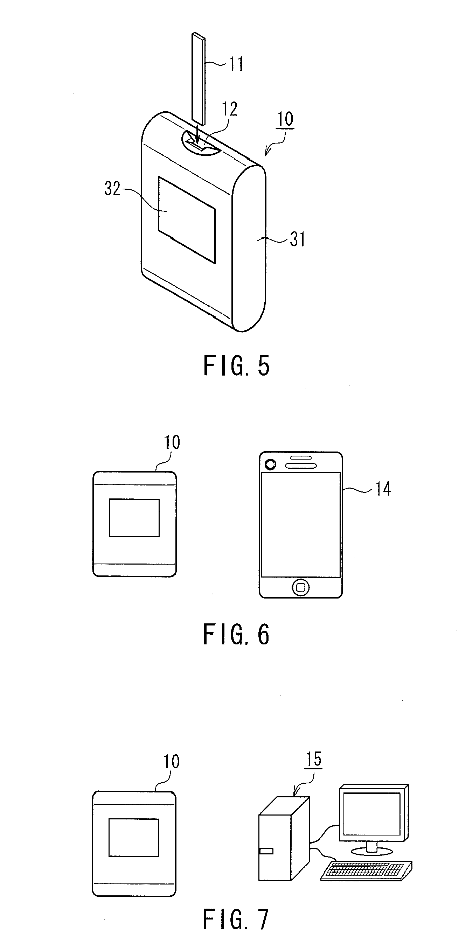

FIG. 5 is a perspective view showing an exemplary blood glucose meter that can be adopted as the measurement device 10. Although in the example shown in FIG. 5, the measurement device 10 is a blood glucose meter, the present invention can be applied also to medical devices for measuring conditions of a living body, such as a sphygmomanometer, a lactic acid meter, a ketone body measurement device, a thermometer, a urine test paper meter, a lipid measurement device, and a pedometer. A main body 31 of the measurement device 10 houses an analysis device and a detector corresponding to the intended use. Further, the measurement device 10 is formed in a palm size, and can be carried by a user, such as a patient, a doctor, a nurse, etc.

As one example, the measurement device 10 can be a portable blood glucose meter that measures blood glucose levels of a patient. In this case, blood of a patient is provided to a sensor 11, and the main body 31 includes a sensor insertion slot 12 for insertion of a strip sensor 11. The sensor 11 has a reagent in its interior, and blood reacts with the reagent inside the sensor 11 beforehand. The main body 31 includes the communication portion 2, the measurement portion 3, the control portion 4, and the memory portion 5 described above. A display screen 32 is one example of the display portion 1. The measurement portion 3 inside the main body 31 has a function of measuring a glucose level from the blood reacted with the reagent by a colorimetric method or an electrochemical method. The measurement by the measurement device 10 starts immediately when the sensor 11 is inserted in the sensor insertion slot 12. The measurement results are recorded in the memory portion 5. The measurement results such as a record including glucose levels and measurement times can be recorded in a file in chronological order. Further, the measurement results are displayed on the display screen 32 of the main body 31. Such a blood glucose meter can be used as an SMBG (Self Monitoring of Blood Glucose) meter, for example.

For example, as shown in FIG. 6, the measurement device 10 can be communicable with a portable device 14, which is an exemplary external device. The portable device 14 can be a smartphone or a tablet terminal with a built-in computer, for example. Thus, data communication between the measurement device 10 and the portable device 14 becomes possible. Further, as shown in FIG. 7, the measurement device 10 and a general-purpose computer 15 (e.g., PC) can be communicable with each other. Examples of the external devices other than the above include a PDA, a portable game machine, and the like, although the applicable external devices are not limited to these examples.

As described above, by applying the measurement device of the above-described embodiment to a medical device such as a blood glucose meter, it is possible to solve problems particular to medical devices. For example, users of portable self-monitoring devices such as the above-described SMBG meter tend to be elderly people, and they often have difficulties in setting wireless communication with external devices by themselves. Hence, simplifying the setting operation is preferred. Further, it is assumed that explanatory meetings may be held or a plurality of measurement devices may be set simultaneously in medical institutions. In that case, a plurality of measurement devices respectively perform communication setting with a plurality of external devices simultaneously in the same place. In this situation, there is a high possibility that erroneous communication is set between a measurement device of a certain user and an external device of another user. There also is a high possibility that, since people who use a blood glucose meter may go to medical institutions at the same time, or have a chance to gather for diabetes camp, the external device and the measurement device under erroneous communication setting may meet again. If the communication setting is made erroneously, the measurement results obtained in another person's measurement device may be transmitted to his/her own external device. In that case, the data processing is executed based on another person's measurement results. Further, his/her own measurement results, which should be treated as personal information, may be displayed on another person's external device.

By the use of the measurement device of the above-described embodiment, problems occurring due to such particular circumstances can be solved. According to the present embodiment, the communication setting is improved, i.e, facilitated. For example, pairing can be established using Bluetooth (registered trademark) version 4.0 without confirmation or input of a device name or a password both in the master device and the slave device. In this case, if two pairs of devices perform a pairing operation at the same timing within an area where radio waves reach, erroneous pairing may be established due to crossing. However, according to the above-described embodiment, confirmation information is transmitted from a measurement device as a slave device, and a master device can judge the appropriateness of the pairing using the confirmation information. Because of this, the data transmitted from the erroneously paired measurement device can be controlled so that the external device cannot use the data even if it receives the data. As a result, even when erroneous pairing is established, it is possible to prevent exchange of data and leakage of personal information.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

### Description of Reference Numerals

- 1, 1a: display portion
- 2, 2a: communication portion
- 3: measurement portion
- 4, 4a: control portion
- 41: setting portion
- 42: transmission and reception portion (transmission portion)
- 41a: measurement device determination portion
- 42a: transmission and reception portion (reception portion)
- 5, 5a: memory portion
- 10: measurement device
- 14: external device

## Claims

1. A medical measurement device that acquires information about a subject body, comprising:
a measurement portion that acquires measurement data obtained by measurement;
a setting portion that specifies an external device as a transmission destination of the measurement data based on a signal transmitted from the external device, and records information of the specified external device, thereby allowing communication with the external device without receiving input of authentication information; and
a transmission portion that transmits the measurement data to the external device specified by the setting portion,
wherein the setting portion causes the transmission portion to transmit identification information of the measurement device for enabling mutual communication with respect to the external device as a transmission source of the received signal, and further to transmit confirmation information thereto in addition to the identification information at or after the transmission of the identification information, the confirmation information being used by the external device to decide whether or not the external device can handle the measurement data transmitted from the measurement device.

2. The measurement device according to claim 1, wherein the setting portion specifies the external device as a transmission destination of the measurement data, and causes the transmission portion to transmit the confirmation information to the external device after being communicable with the external device.

3. The measurement device according to claim 1 or 2, wherein the setting portion causes a display portion of the measurement device to display the confirmation information.

4. The measurement device according to any one of claims 1 to 3,
wherein the setting portion causes the transmission portion to transmit the confirmation information to the external device in association with measurement data to be transmitted by the transmission portion, and
the confirmation information is information to be used by the external device to decide the usability of the measurement data in the external device.

5. The measurement device according to any one of claims 1 to 4, wherein the setting portion cancels a state communicable with the external device when receiving a notification from the external device that the external device has judged the measurement data as not handleable based on the confirmation information.

6. A medical measurement system, comprising:
a medical measurement device that acquires information about a subject body; and
an external device,
wherein the measurement device includes:
a measurement portion that acquires measurement data obtained by measurement;
a setting portion that specifies an external device as a transmission destination of the measurement data based on a signal transmitted from the external device, and records information of the specified external device, thereby allowing communication with the external device without receiving input of authentication information; and
a transmission portion that transmits the measurement data to the external device specified by the setting portion, and
the external device includes:
a measurement device determination portion that specifies a measurement device as a transmission source of the measurement data based on a signal from the measurement device, and allows communication with the measurement device without receiving input of authentication information; and
a reception portion that receives the measurement data from the specified measurement device,
wherein the setting portion of the measurement device causes the transmission portion to transmit identification information of the measurement device for enabling mutual communication with respect to the external device as a transmission source of the received signal, and further to transmit confirmation information thereto in addition to the identification information at or after the transmission of the identification information, and
the measurement device determination portion of the external device decides whether or not the external device can handle the measurement data transmitted from the measurement device based on the confirmation information transmitted from the measurement device.

7. A method performed by a medical measurement device that acquires information about a subject body, the method comprising:
acquiring measurement data obtained by a measurement portion of the medical measurement device;
specifying an external device as a transmission destination of the measurement data based on a signal transmitted from the external device;
recording information of the specified external device, thereby allowing communication with the external device without receiving input of authentication information; and
transmitting the measurement data to the external device specified by the setting portion,
wherein the specifying causes the transmitting to transmit identification information of the measurement device for enabling mutual communication with respect to the external device as a transmission source of the received signal, and further to transmit confirmation information thereto in addition to the identification information at or after the transmission of the identification information, the confirmation information being used by the external device to decide whether or not the external device can handle the measurement data transmitted from the measurement device.

8. The method according to claim 1, wherein the specifying specifies the external device as a transmission destination of the measurement data, and causes the transmitting to transmit the confirmation information to the external device after being communicable with the external device.

9. The method according to claim 7 or 8, wherein the specifying causes a display portion of the measurement device to display the confirmation information.

10. The method according to any one of claims 7 to 9,
wherein the specifying causes the transmitting to transmit the confirmation information to the external device in association with measurement data to be transmitted by the transmitting step, and
the confirmation information is information to be used by the external device to decide the usability of the measurement data in the external device.

11. The method according to any one of claims 7 to 10, wherein the specifying cancels a state communicable with the external device when a notification is received from the external device that the external device has judged the measurement data as not handleable based on the confirmation information.

12. A method performed by an external device that is arranged to communicate with the measurement device of any of claims 1 to 5, the method comprising:
specifying a measurement device as a transmission source of measurement data based on a signal from the measurement device, and allowing communication with the measurement device without receiving input of authentication information; and
receiving the measurement data from the specified measurement device,
wherein the receiving receives identification information of the measurement device for enabling mutual communication with respect to the measurement device and receives confirmation information in addition to the identification information at or after reception of the identification information, and
deciding whether or not the external device can handle the measurement data transmitted from the measurement device based on the confirmation information received from the measurement device.

13. An external device for use with the measurement device of any of claims 1 to 5, the external device comprising:
a measurement device determination portion that specifies a measurement device as a transmission source of the measurement data based on a signal from the measurement device, and allows communication with the measurement device without receiving input of authentication information; and
a reception portion that receives the measurement data from the specified measurement device,
wherein the reception portion is arranged to receive identification information of the measurement device for enabling mutual communication with respect to the measurement device and to receive confirmation information in addition to the identification information at or after reception of the identification information, and
wherein the measurement device determination portion is arranged to decide whether or not the external device can handle the measurement data transmitted from the measurement device based on the confirmation information transmitted from the measurement device.

14. A computer implementable instructions product comprising computer implementable instructions for causing a programmable device to perform the method of any of claims 7 to 12.
